# EUROPEAN PATENT APPLICATION

(11) **EP 2 636 775 A1**
(43) Date of publication of application: **11.09.2013**
(21) Application number: 11837488.3
(22) Date of filing: 09.08.2011
(51) Int. Cl.: D01F 1/10, D01F 2/00, D01F 9/04, D01F 9/00, D01D 1/02, D03D 1/00, A61L 15/46

(54) **ANTIMICROBIAL FIBER, FABRIC AND WOUND DRESSING CONTAINING NANO METAL AND PREPARATION METHOD THEREOF**

(30) Priority: 03.11.2010 CN 201010530059
(71) Applicant: Guangdong Baihe Medical Technologies Ltd, Foshan, Guangdong 528225 (CN)
(72) Inventor: WANG, Xiaodong, Foshan Guangdong 528225 (CN); WANG, Rui, Foshan Guangdong 528225 (CN); CEN, Rongzhang, Foshan Guangdong 528225 (CN); MO, Xiaohui, Foshan Guangdong 528225 (CN)
(74) Representative: Hellmich, Wolfgang
(86) International application number: PCT/CN2011/078147
(87) International publication number: WO 2012/058962

(57) **Abstract**

The present invention discloses an antimicrobial fibre, fabric and wound dressing that incorporate nano metal and the preparation method thereof. Nano metal particles are homogenously distributed within the fibre structure or surface, and, the fibre does not contain protective colloid for the prevention of agglomeration of nano metal particles. The nano metal particles are in an amount of 1.1 % to 20% by weight. The fibrous wound dressing has the ability to continuously release sufficient amount of silver ions and is especially suitable for the management of chronic wounds. It can effectively prevent various bacteria and microbial infections.

## Description

### FIELD OF THE INVENTION

The present invention involves an antimicrobial fibre, a fabric, and a wound dressing containing nano metal and its preparation method.

### BACKGROUND OF THE INVENTION

It is well known that silver and copper, particularly silver, have been recognised for a long time for their effective antimicrobial properties, particularly for killing some bacteria found commonly in the management of chronic wounds. More importantly silver has demonstrated its effectiveness against some antibiotic-resistant bacteria.

At present, there are two types of silver antimicrobial wound dressing, one is ionic and the other is metallic. Ionic technology employs a silver compound in the manufacture of the wound dressing. Metallic nano silver technology uses nano metal, e.g. nano silver particles in the manufacture of the wound dressing.

There are two methods in the manufacturing of antimicrobial fibrous wound dressing. One is to add silver particles into the structure of fibre compounds, i.e. to blend the silver material into the spinning solution and allow the antimicrobial agent remain within the fibre.

US 6897349 and EP 1216065 disclosed a method of preparing a silver antimicrobial material. This method is to disperse silver ions into a hydrophilic polymer matrix during the fibre preparation process.

CN1308509 disclosed a silver chitosan fibre with antimicrobial function and its preparation method. This method is to mix silver sodium hydrogen zirconium phosphate (trade name Alphasan) with particle size of about 1 micron into spinning solution. The content of silver compounds is 3.0% to 4.0% w/w.

CN 1060235C disclosed a bacteriostatic acrylic fibre and its preparation method. This method is to blend the antimicrobial master batch into the polypropylene and to spin into fibre. The fibre contains 500pmm to 1000ppm (w/w) of bacteriostatic agent. The second method is applying the antimicrobial agent on the surface of the fibre or the fabric, i.e. permeating or absorbing onto the surface of the fibre or fabric.

CN 1895683A disclosed a nano silver antimicrobial dressing and its preparation method. This method is to coat the nano silver solution onto the fabric to form a dressing with 0.05% to 2.9% w/w of nano silver.

CN 100346840C disclosed a composite nano medical dressing, the invention is to composite nano silver particles, of size 1nm to 15nm, onto a nonwoven or carbon fibre absorbing materials.

CN 1066783A disclosed a method of making an antimicrobial material employing an antimicrobial metal. The method is to form an antimicrobial material containing antimicrobial metals, such as silver, copper, etc., or their alloy by physical method, e.g. vapour deposition.

US 7462753 disclosed a nano silver wound dressing with 4 layers in structure, the first was a hydrophilic fabric; the second layer was formed by an activated charcoal fabric impregnated with nanocrystalline silver; the third layer was a superabsorbent nonwoven and the forth layer was porous fabric to cover the third layer.

EP 1095179 disclosed a method of making nonwoven fabric used for wound dressing. The method employed a lamination technique to combine silver coated nylon scrim with an alginate fabric on both sides of the silver coated nylon scrim.

US7385101 disclosed an antimicrobial textile material used for wound dressing and the wound dressing itself. It blended silver coated textile fibres with alginate fibres in the nonwoven process to achieve the antimicrobial wound dressing.

US 20030180346 and EP1318842 described a silver wound dressing by blending a silver fibre and a non silver fibre, there are 0.01 % to 5.0% w/w of the silver ion in the finial wound dressing.

EP 1849464 and US 2007275043 disclosed a method of adding a silver compound (silver carbonate) into the fibres, i.e. to mix the silver compound into the spinning dope.

CN 1673425A explained a method of making an antimicrobial viscose fibres containing 0.1 % to 1% w/w of nano silver which is added into the spinning dope. However, this method has to use a protective colloid agent with the maximum amount of 2% w/w. All the protective colloid agent exists inside of the fibres in the form of suspension but not as a part of crosslink with the rest of molecules of the fibres. This method actually reduced the proportion of polymer compounds within the fibres, and restricted the amount of nano silver that could be possibly included in the fibre. In fact this method only allowed a maximum 1% w/w of nano silver material by weight..

It can be seen, the method of adding silver ions as antimicrobial agent into the wound dressing can be either to blend the silver compounds into the spinning dope and to allow them to be retained in the finished fibre, or to apply the antimicrobial agent onto the surface of fibres or fabrics and allow them to be permeated or absorbed on the surface of fibres or fabrics. But in general, the silver content is limited in silver compounds; therefore a large amount of silver compound has to be used to achieve the required silver content within the fibres. However the proportion of polymer in the fibre structure has to be reduced to give space to the amount of silver compound, therefore the fibres become less strong, soft and hydrophilic. In order to maintain the fibre strength, the amount of silver compound is normally controlled to less than 10% w/w of the fibre polymer, thus the resultant silver content is very small. Therefore the silver content of the dressing is very small too, Consequently the performance and lifetime of an antimicrobial wound dressing is restricted. But in fact the clinical application requires such a dressing to deliver a much more powerful and durable performance, for example for the treatment of burns, a good antimicrobial wound dressing is expected to be used continuously for up to 7 or 21 days. Furthermore, in the available technologies, metal silver particles or even nano silver particles were added onto the surface of fibres or fabrics by padding, impregnating or vapour deposition. The silver particles were only absorbed onto the surface of fibres and fabrics, it was therefore difficult to achieve a high silver content. Moreover, as the silver particles only exist on the surface of fibre, the silver particles may come off of the fibre by the factors of surface moisture, gel formation or softening, etc., further limiting its capability of releasing antimicrobial agents to the wound sites.

The present invention provides such a solution to above problems by mixing the nano metal particles, preferably nano silver particles, directly into the spinning solution (as known as dope). With the help of the certain viscosity of the spinning dope, the nano metal particles are suspended in the dope rather than agglomerated. Thereby the nano metal particles are homogenously distributed in the fibre structure. The maximum nano metal content that can be achieved is 20% w/w.

Other than using of the protective colloid agent which reduces the proportion of the polymer in the fibres, this invention makes the nano metal particles suspended rather than agglomerated by utilising the viscosity of the spinning dope. It makes it possible to add large amounts of nano metal particles into the spinning dope, thereby producing an effective antimicrobial fabric and dressing to achieve long lasting antimicrobial effects of up to 7 days.

Therefore, the purpose of the present invention is to use a high content of nano metal particles homogenously distributed in the fibre structure in the manufacture of antimicrobial fibre, fabric and wound dressing.

### SUMMARY OF THE INVENTION

The present invention provides an antimicrobial fibre, an antimicrobial fabric and an antimicrobial wound dressing and their preparation method.

Because the nano particles involved in this invention are generally in the range of 5 to 10nm, it only occupies one thousandth of the fibre diameter. Unlike the other available technologies (e.g. CN1308509C and EP1849464A1), the dimension of the silver compounds particle takes 5% to 10% of the fibre diameter; causing weakness in the fibre structure and physical performance. More importantly, because the nano silver particles are added into spinning polymer solution prior to extrusion, it can be evenly distributed into the structure of the formed fibre. Once the antimicrobial wound dressing containing nano metal contacts with the wound exudate, the nano metal particles will begin releasing metal ions into the dressing fibres while the particles themselves are still kept in the fibre structure. The nano metal particulars in the fibre structure can provide continuous release of metal ions allowing a more durable antimicrobial function.

In the present invention, the content of nano particles in the dressing is between 1.1 % to 20% w/w, preferably 1.2% to 18% w/w, most preferably 1.5% to 15% w/w.

The size of nano metal particles used in the present invention can be between 1nm and 1000nm, preferred size is 1 nm to 400nm.

The nano metal used in the present invention can be silver, copper or zinc.

The fibres used in the dressing involved in the present invention can be alginate fibre, chitosan fibre or cellulose fibre (including solvent spun cellulose fibre). The said alginate fibre can be a high M (mannuronic acid), high G (guluronic acid) or a M/G mixed alginate fibre. The said alginate fibre can be a calcium alginate or calcium/sodium alginate fibre. The said chitosan fibre can be one with a minimum 80% w/w deacetylation. The said chitosan fibre containing nano metal can be further treated chemically to convert into a gel forming fibre. The said viscose fibre can also be a traditional viscose fibre or a solvent spun cellulose fibre. The said cellulose fibre containing nano metal can be further treated chemically to convert into gel forming fibre.

The fibre used in the present invention can be staple fibre, the fibre length can be such that suit the need for process and the final products, typically between 3mm and 100mm.

The fibre used in the present invention shall have certain of linear density and crimp. The fibre linear density shall be between 1 dtex and 5 dtex, preferrably between 1.5 dtex and 3 dtex.

The dressing involved in the present invention can be made through the needling nonwoven process, the chemical bonding process, weaving or the knitting processes. If the needling process is employed, the fibre length can be longer, between 30mm to 100mm. If the chemical bonding process is used, the fibre length can be shorter, between 3mm to 15mm. If weaving or knitting is employed, the fibre length can be between 20mm to 85mm.

Unlike other technology that employs a protective colloid agent causing reduction of percentage of polymer in the fibre, in this invention, the nano particles are suspended evenly in the polymer solution by the high viscosity of the spinning dope. It therefore becomes possible to add a large quantity of nano metal particles into the spinning dope, thereby producing a more effective antimicrobial fibre and dressing to achieve a long term antimicrobial effect.

The present invention provides a method to prepare an antimicrobial fibre that contains nano metal. The details of such a method are: Firstly dissolve a part of spinning polymer in a mixing tank, the volume of the pre-dissolved spinning polymer shall be such that the viscosity of the polymer solution in the mixing tank is between 500 and 1000 centipoise to prevent the nano particles from re-agglomeration, this will allow the nano metal particles to be evenly distributed into the spinning polymer then into the fibres. Secondly add the nano metal particles into the pre-mixed polymer solution. If the nano metal particles are in powder form, a low frequency ultrasound can be used to assist in the dispersion of nano particles into a nano metal aqueous solution, and then add the aqueous solution into the mixing tank. Finally, add all remaining polymer into the mixing tank following the normal mixing procedures.

The finial spinning dope shall have sufficient viscosity, e.g. 3000 centipoise, or above. Otherwise the nano metal particles will become agglomerated or precipitated to the bottom of mixing tank. This will result in nano metal particles being unevenly distributed into the fibre structure.

The present invention provides a method for the preparation of an antimicrobial fibre and wound dressing that contains nano metal. The method has following steps:
Disperse 1.1% to 20% w/w of nano metal particles into the spinning dope solution,
and convert into fibres by wet spinning technology.

Convert the obtained fibres into fabrics by nonwoven, weaving or knitting technology.

The present invention provides a method to prepare an antimicrobial wound dressing that contains nano metal including following steps: cut the obtained fabrics, pack and sterilise to obtain the mentioned dressing.

The said nano metal particles involved in above method can be nano silver, nano copper or nano zinc particles.

The said nano metal particles involved in above method shall have a size range of between 1 nm and 1000nm, preferably 1 nm to 400nm.

Because the wound dressing involved in the present invention is composed of fabrics that contain fibres with nano metal particles both in the structure and on the surface of fibre, the said nano metal particles are evenly distributed through the cross section of the fibre. Therefore, the wound dressing has an ability to release sufficient amount of metal ions on contact with wound exudates. This ability is particularly beneficial to chronic wound care. It can provide a durable and effective antimicrobial function.

### BRIEF DESCRIPTION OF THE DRAWINGS

Fig. 1 shows the Zone of Inhibition at 1 day against staphylococcus aureus of a 5% w/w nano silver dressing;
Fig. 2 shows the Zone of Inhibition at 7 day against staphylococcus aureus of a 5% w/w nano silver dressing;
Fig. 3 shows the Zone of Inhibition at 1 day against escherichia coli of a 1.5% w/w nano copper dressing;
Fig. 4 shows the Zone of Inhibition at 7 day against escherichia coli of a 1.5% w/w nano copper dressing;
Fig. 5 shows the Zone of Inhibition at 1 day against staphylococcus aureus of a 1.1 % w/w nano silver dressing;
Fig. 6 shows the Zone of Inhibition at 7 day against staphylococcus aureus of a 1.1 % w/w nano silver dressing;
Fig. 7 shows the Zone of Inhibition at 1 day against pseudomonas aeruginosa of a 15% w/w nano silver dressing;
Fig. 8 shows the Zone of Inhibition at 7 day against pseudomonas aeruginosa of a 15% w/w nano silver dressing;
Fig. 9 shows the Zone of Inhibition at 1 day against staphylococcus aureus of a 15% w/w nano silver dressing; and
Fig. 10 shows the Zone of Inhibition at 7 day against staphylococcus aureus of a 15% w/w nano silver dressing.

### DETAILED DESCRIPTION OF THE PREFERRED EMBODIMENTS

The present invention can be further illustrated through the following examples.

### Example 1

An antimicrobial wound dressing containing nano silver particle is produced by the following steps:
1. Charge the mixing tank with a specified quantity of water;
2. Work out the quantity of sodium alginate powder according to the moisture content of the alginate powder and required solid content. For example, if the sodium alginate has a moisture content of 10% and the required solid content of the spinning dope solution is 5%, for 20 kg of sodium alginate powder, 342 litres of water is needed.
3. Work out the quantity of nano silver particle required. For a product containing 0.5% of silver by weight (the moisture content of the nano silver is ignored) and the dry mass of the sodium alginate is 18 kg, about 0.09 kg of nano silver is needed.
4. Prepare a silver solution containing 20% nano silver particle (5 nm, D₉₅ = 3.7-6.9). The water contained in the nano silver aqueous solution shall come from the volume calculated according to Step 2. An ultrasound can used to facilitate the dispersion of the nano solution.
5. Add 4 kg of sodium alginate powder into the tank that has been previously charged with specified quantity of water (above steps 1 and 2). Add the nano silver solution into the mixing while keeping the mixer on in order to disperse the nano silver particles evenly.
6. While the mixer is running, the remaining sodium alginate powder can be slowly added into the mixer.
7. On dispersing all the sodium alginate, the mixer can be taken out and the mixing tank can be left to stand for degassing. This normally takes about 24 hrs. Because of the high viscosity of the dope solution, the nano silver particles are suspended in the dope solution.
8. Once most of the air bubbles disappear from the dope solution, all the dope can be spun into fibres following normal extrusion procedures for a calcium alginate fibre, i.e. through a number of wet spinning steps such as the extrusion bath to convert sodium alginate dope into calcium alginate fibre, the orientation bath and the haul off rollers to align the molecular chains, the washing/drying/crimping and cutting to make the fibre more suitable for the carding process.
9. The finished fibre is slightly off white in colour and shall have about 0.5% w/w of silver content.
10. Convert the fibre made from this process into a nonwoven felt using traditional carding and needling processes. The finished felt is cut into 10x10 cm and packed into a paper pouch. The dressing is sterilised by a gamma irradiation process at a dosage of 25-40 kGy.
11. Completion of all above steps will obtain an antimicrobial wound dressing containing 5% w/w of nano silver.

### Example 2

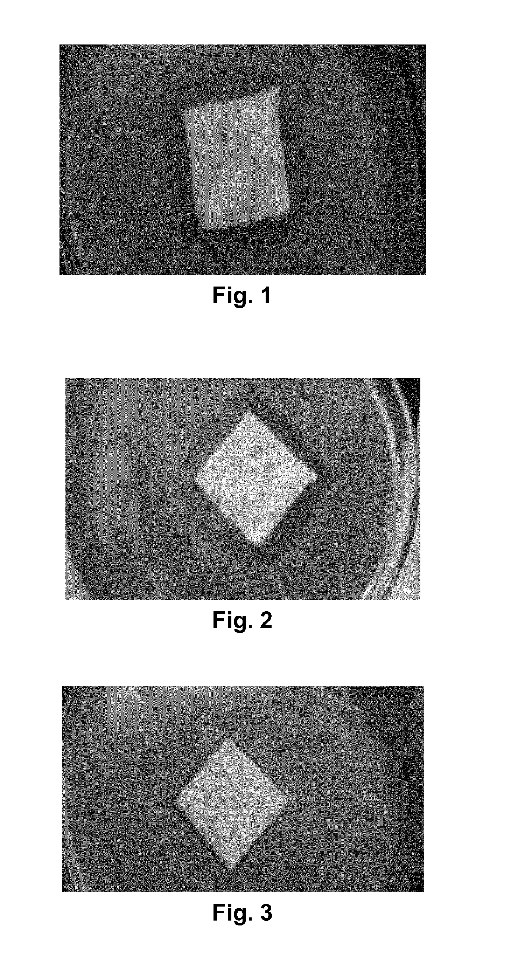

In order to observe the antimicrobial performance of the dressing, a fixed amount of staphylococcus aureus was evenly coated on a petri dish. Then the dressing obtained from Example 1 was cut into 2x2 cm and put into the petri dish. The petri dish was then cultured for 7 days at temperature of 37 °C, and observed daily for the growth of bacteria on the plate. The silver ions released from the dressing kill the bacteria around the dressing forming a zone of inhibition and this zone of inhibition is clearly visible. Fig. 1 shows the zone of inhibition at day1, and Fig. 2 shows the zone of inhibition at day 7. It can be seen that 5% w/w of nano silver dressing has a good antimicrobial performance after seven days.

### Example 3

The method of preparing an antimicrobial wound dressing containing 1.5% w/w nano copper:
1. Charge the mixing tank with a specified quantity of water;
2. Work out the quantity of sodium alginate according to the moisture content of the alginate powder and the required solid content. For example, if the sodium alginate has a moisture content of 10% and the required solid content of the spinning dope solution is 5%, for 20 kg of sodium alginate powder, 342 litres of water is needed. The dry mass of the sodium alginate powder is 18 kg.
3. Prepare the nano copper solution, the nominal particle size is 400nm, the actual size range is between 300nm to 550nm.
4. For a product containing 1.5% w/w of copper and the 18 Kg dry mass of the sodium alginate, about 1.35 kg of 20% w/w of nano copper solution is needed.
5. Add 4 kg of sodium alginate powder into the tank containing the specified quantity of water (step 1 and 2 above). After dissolving all the sodium alginate, add in the above nano copper solution. Keep the mixer running throughout the process.
6. With the mixer running, add in the remaining sodium alginate powder slowly into the mixer.
7. On dispersing all the sodium alginate, the mixer can be taken out and the mixing tank can be left to stand for degassing. This normally takes about 24 hrs.
8. Once most air bubbles disappear from the dope solution, the said dope can be spun, following normal extrusion procedures, into a calcium alginate fibre containing nano copper.
9. Convert the fibre made from this process into a nonwoven felt using traditional carding and nonwoven process. The finished felt is cut into 10x10 cm and packed into a paper pouch. The dressing is sterilised by a gamma irradiation at a dosage of 25-40 KGy.
11. Completion of all above steps will obtain an antimicrobial wound dressing containing 1.5% w/w nano copper.

### Example 4

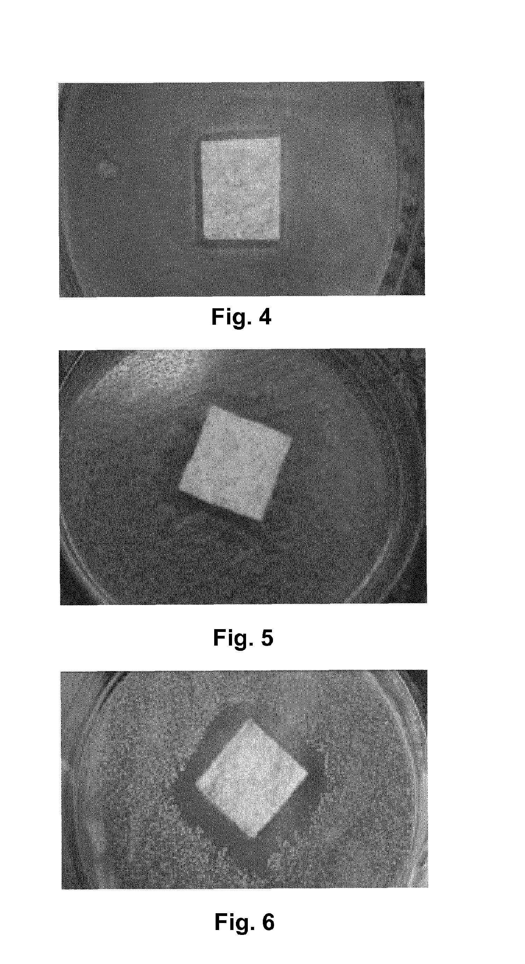

In order to observe the antimicrobial performance of the dressing, a fixed amount of escherichia coli was evenly coated onto a petri dish. The dressing obtained from the Example 3 was cut into 2x2 cm and put into the petri dish. The sample and the petri dish were cultured for 7 days at temperature of 37 °C, and observed daily for the growth of bacteria on the plate. The copper ions released from the dressing kill the bacteria around the dressing forming a zone of inhibition, which is clearly visible. Fig. 3 shows the zone of inhibition at day 1, and Fig. 4 shows the zone of inhibition at day 7. It can be seen that the said nano copper dressing has a good antimicrobial performance after seven days.

### Example 5

The method of preparing an antimicrobial chitosan wound dressing containing 1.1% w/w nano silver:
1. Work out the quantity of chitosan according to the moisture of the chitosan powder and required solid content. For example, if the chitosan powder has a moisture content of 10% and required solid content of the spinning dope solution is 5%, for 200 g of chitosan powder, 342 millitres of 2% acetic acid aqueous solution is needed. The dry mass of chitosan powder is 180 g.
2. Work out the quantity of nano silver powder required. For a product containing 1.1 % of silver by weight (the moisture content of the nano silver is ignored) and the dry mass of the chitosan powder is 180 g, about 1.98 g of nano silver is needed.
3. Prepare a silver solution containing 20% nano silver particles (5 nm, D₉₅ = 3.7-6.9). The water contained in the nano silver aqueous solution shall come from the volume calculated according to Step 2. An ultrasound can used to facilitate the dispersion of the nano solution.
4. Add 30 g of chitosan powder into the tank containing aforementioned acetic acid aqueous solution. Then add the nano silver solution into the mixed chitosan solution. Keep the mixer on during this process to ensure that the nano particles are fully dispersed.
5. While the mixer is running, add the remaining chitosan powder into the mixer.
6. On dispersing all the chitosan, the mixer can be taken out and the mixing tank can be left to stand for degassing..
7. Once most air bubbles disappear from the dope solution, all the dope can be spun into chitosan fibre following normal extrusion procedures.
8. The finished fibre is pale yellow in colour and shall have about 1.1 % w/w of silver content.
9. Convert the fibre made from this process into a nonwoven felt using traditional carding and needling process. The finished felt is cut into 10x10 cm and packed into a paper pouch. The dressing is sterilised by a gamma irradiation process at dosage of 25-40 kGy.
10. Completion of all above steps will obtain nano silver chitosan wound dressing.

### Example 6

In order to observe the antimicrobial performance of the dressing, a fixed amount of staphylococcus aureus was evenly coated onto a petri dish. Then the dressing obtained from the Example 5 was cut into 2x2 cm and put into the petri dish. The petri dish was cultured for 7 days at temperature of 37 °C, and observed daily for the growth of bacteria on the plate. Fig. 5 shows the Zone of Inhibition at day 1. Fig. 6 shows the Zone of Inhibition at day 7. It can be seen that the said nano silver chitosan dressing has good antimicrobial performance after seven days.

### Example 7

The method of preparing an antimicrobial wound dressing containing 15% w/w nano silver through a wet spinning process:
1. Charge the mixing tank with a specified quantity of water;
2. Work out the quantity of sodium alginate powder according to the moisture content of the alginate powder and required solid content. For example, if the sodium alginate has a moisture content of 10% and the required solid content of the spinning dope solution is 5%, for 200 g of sodium alginate powder, 3.42 litres of water is needed. The dry mass of the sodium alginate is 180 g.
3. Work out the quantity of nano silver particle required. For a product containing 15% of silver by weight (the moisture content of the nano silver is ignored) and the dry mass of the sodium alginate is 180 g, about 27 g of nano silver (5nm, D₉₅ = 3.7-6.9) is needed.
4. Prepare a silver solution containing 40% nano silver particles. The water contained in the nano silver aqueous solution shall come from the volume calculated according to Step 2. An ultrasound can used to facilitate the dispersion of the nano solution.
5. Add 30g of sodium alginate powder into the tank that has been previously charged with specified quantity of water (above steps 1 and 2). Add the nano silver solution into the mixing while keeping the mixer on in order to make the nano silver particles dispersed evenly.
6. While the mixer is running, the remaining sodium alginate powder can be slowly added into the mixer.
7. On dispersing all sodium alginate, the mixer can be taken out and the mixing tank can be left to stand for degassing.
8. Once most of the air bubbles disappear from the dope solution, all the dope can be spun into fibres following normal extrusion procedures for a calcium alginate fibre, i.e. through a number of wet spinning steps such as the extrusion bath to convert sodium alginate dope into calcium alginate fibre, the orientation bath and the haul off rollers to align the molecular chains, the washing/drying/crimping and cutting to make the fibre more suitable for the carding process.
9. The finished fibre is slightly off white in colour and shall have about 15% w/w of silver content.
10. Convert the fibre made from this process into a nonwoven felt using traditional carding and needling processes. The finished felt is cut into 10x10 cm and packed into a paper pouch. The dressing is sterilised by a gamma irradiation process at a dosage of 25-40 kGy.
11. Completion of all above steps will obtain a silver alginate wound dressing containing 15% nano silver particles.

### Example 8

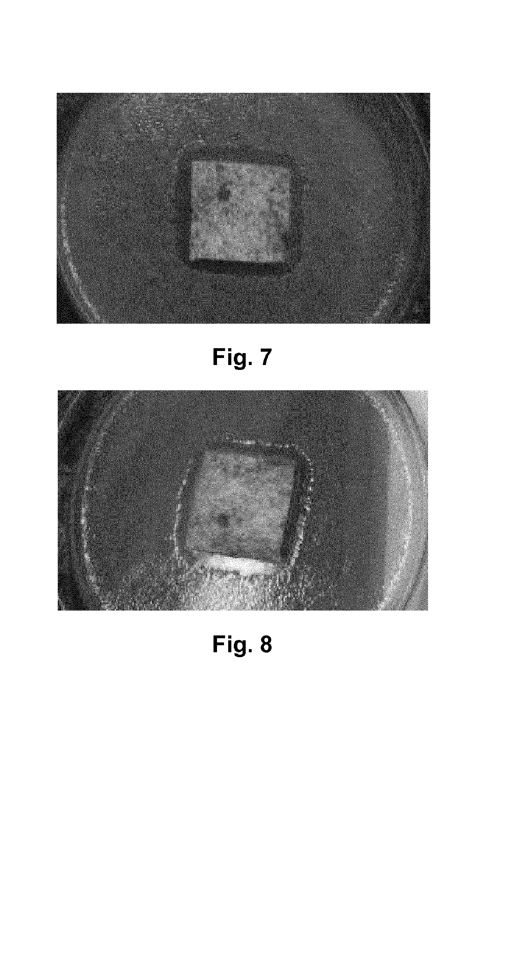

In order to observe the antimicrobial performance of the dressing, a fixed amount of pseudomonas aeruginosa was evenly coated onto a petri dish. Then the dressing obtained from the Example 7 was cut into 2x2 cm and put into the petri dish. The petri dish was cultured for 7 days at temperature of 37 °C, and observed daily for the growth of bacteria on the plate. Fig. 7 shows the Zone of Inhibition at day one. Fig. 8 shows the Zone of Inhibition at day seven. It can be seen that the said nano silver dressing has a good antimicrobial performance after seven days.

### Example 9

In addition to nano metal fibre, the nano metal polymer film can also be produced using a similar principle. The following method is employed for the preparation of a calcium alginate film containing nano silver. The process is similar to that of silver alginate fibre, the only difference is that the product is a film instead of fibre and that there is no orientation, washing and drying process.
1. Charge the mixing container with a specified quantity of water;
2. Work out the quantity of sodium alginate according to the moisture content of the alginate powder and the required solid content. For example, if the sodium alginate has a moisture content of 10% and the required solid content of the alginate dope solution is 5%, for 200 g of sodium alginate powder, 3.42 litres of water is needed. The dry mass of the sodium alginate is 180 g.
3. Work out the quantity of nano silver powder required. For a product containing 20% of silver by weight (the moisture content of the nano silver is ignored) and the dry mass of the sodium alginate is 180 g, about 36 g of nano silver is needed.
4. Prepare a silver solution containing 40% nano silver particles. The water contained in the nano silver aqueous solution shall come from the volume calculated according to Step 2. An ultrasound can used to facilitate the dispersion of the nano solution.
5. Add 30g of sodium alginate powder into the tank that has been previously charged with specified quantity of water (above steps 1 and 2). Add the nano silver solution into the mixing while keeping the mixer on in order to dispere the nano silver particles evenly.
6. While the mixer is running, the remaining sodium alginate powder can be slowly added into the mixer.
7. The mixed alginate and silver solution can be left tostand still for 30 minutes, then evenly spread on a flat plate. The layer thickness shall be between 0.5 and 1.0 mm. Then place the plate and the sodium alginate layer into a container filled with calcium chloride solution, ensuring that the sodium alginate is fully covered by the calcium chloride solution. A thin layer of alginate containing nano silver particles is formed. After 5 mins, take out the film and leave it in a suitable place to dry.
8. The finished film has 20% w/w of nano silver content and is slightly black in colour caused by the high content of nano silver.
9. The finished film is cut into 10x10 cm and packed into a paper pouch. The dressing is sterilised by a gamma irradiation process at a dosage of 25-40 kGy.
10. This will obtain a silver alginate film dressing containing 20% w/w of nano silver.

### Example 10

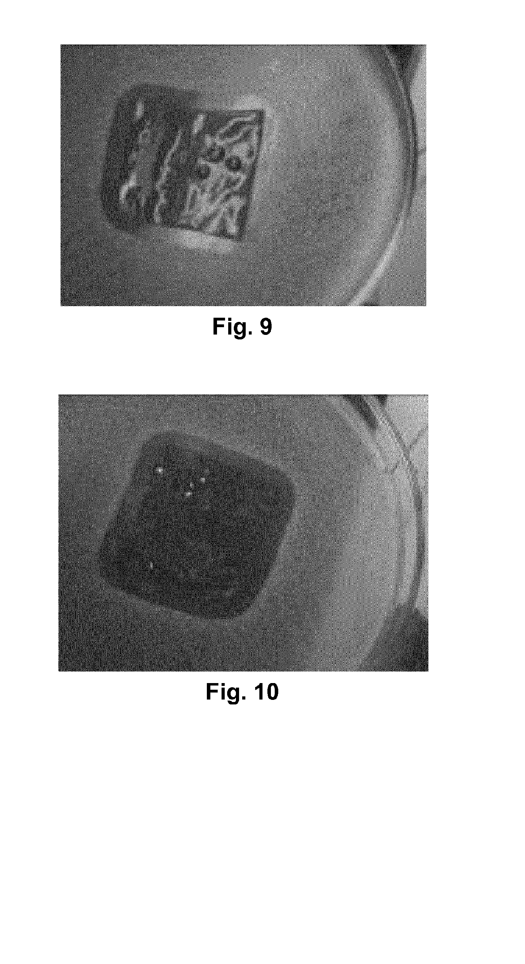

In order to observe the antimicrobial performance of the dressing, a fixed amount of pseudomonas aeruginosa was evenly coated onto a petri dish. Then the dressing obtained from the Example 9 was cut into 2x2 cm and put into the petri dish. The petri dish was cultured for 7 days at temperature of 37 °C, and observed daily for the growth of bacteria on the plate. Fig. 9 shows the Zone of Inhibition at day one. Fig. 10 shows the Zone of Inhibition at day 7. It can be seen that 20% w/w of nano silver film also has good antimicrobial performance after seven days.

## Claims

1. An antimicrobial fibre containing nano metal wherein nano metal particles are homogenously distributed in the fibre structure or surface, and there does not contain protective colloid agent in the fibre to prevent agglomeration of the nano metal particles.

2. The antimicrobial fibre containing nano metal according to claim 1, wherein the content of the nano metal particles is between 1.1 % to 20% w/w, preferably 1.2% to 18% w/w, most preferably 1.5% to 15% w/w.

3. The antimicrobial fibre containing nano metal according to claim 1 or 2, wherein the nano metal particles have a dimension of 1nm to 1000nm, preferably 1 nm to 400nm.

4. The antimicrobial fibre containing nano metal according to claim 1 or 2, wherein the nano metal particles is nano silver, nano copper or nano zinc particles.

5. The antimicrobial fibre containing nano metal according to claim 1 or 2, wherein the fibre is an alginate, a chitosan or a viscose fibre.

6. The antimicrobial fibre containing nano metal according to claim 5, wherein the alginate fibre is a high M (mannuronic), a high G (guluronic) or a M/G mixed alginate fibre.

7. The antimicrobial fibre containing nano metal according to claim 5, wherein the alginate fibre is a calcium alginate or a calcium/sodium alginate fibre.

8. The antimicrobial fibre containing nano metal according to claim 1 or 2, wherein the fibre length is between 3mm to 100mm.

9. The antimicrobial fibre containing nano metal according to claim 1 or 2, wherein the fibre linear density is between 1 dtex to 5 dtex, preferably 1.5 dtex to 3 dtex.

10. An antimicrobial fabric, wherein the antimicrobial fabric is made of the antimicrobial fibres containing nano metal of claim 1.

11. A method of preparing antimicrobial fibres containing nano metal of claim 1, comprising:
pre-mixing a low viscosity spinning dope with a viscosity of between 500 and 1000 centipoise, adding nano metal solution into the dope; wherein the content of the nano metal particles is between 1.1 % to 20% w/w, preferably 1.2% to 18% w/w, the most preferably 1.5% to 15% w/w.

12. The method of preparing antimicrobial fibres containing nano metal according to claim 11, wherein the fibre is an alginate, a chitosan or viscose fibre.

13. The method of preparing antimicrobial fibres containing nano metal according to claim 12, wherein the alginate fibre is high M (mannuronic), high G (guluronic) or M/G mixed alginate fibre.

14. The method of preparing antimicrobial fibres containing nano metal according to claim 12 or claim 13, wherein the alginate fibre is a calcium alginate or a calcium/sodium alginate fibre.

15. The method of preparing antimicrobial fibres containing nano metal according to claim 11, wherein the nano metal is nano silver, nano copper or nano zinc particles.

16. An antimicrobial fibrous wound dressing containing nano metal, wherein the antimicrobial fibrous wound dressing is made from the fabric of claim 10.

17. A method of preparing an antimicrobial fibrous wound dressing containing nano metal of claim 16, wherein the method includes following process steps:
dispersing the nano metal particles into the spinning dope solution at the nano metal content of 1.1 % to 20% w/w, preferably 1.2% to 18% w/w, most preferably 1.5% to 15% w/w;
converting the spinning dope solution into a fibre by the wet spinning process;
converting the obtained fibres into fabrics through the nonwoven, weaving or knitting process; and
cutting, packing and sterilising the fabrics into wound dressing.

18. The method of preparing an antimicrobial fibrous wound dressing containing nano metal according to claim 17, wherein the nano metal particles are nano silver, nano copper or nano zinc particles.

19. The method of preparing an antimicrobial fibrous wound dressing containing nano metal according to claim 18, wherein the nano metal particles have a dimension of 1 nm to 1000nm, preferably 1 nm to 400nm.

20. The method of preparing an antimicrobial fibrous wound dressing containing nano metal according to claim 17, wherein the fibre is alginate, chitosan or viscose fibre.

21. The method of preparing an antimicrobial fibrous wound dressing containing nano metal according to claim 17, wherein the alginate fibre is high M (mannuronic), high G (guluronic) or M/G mixed alginate fibre.

22. The method of preparing an antimicrobial fibrous wound dressing containing nano metal according to claim 20 or 21, wherein the alginate fibre is calcium alginate or calcium/sodium alginate fibre.
